Europäisches Patentamt

European Patent Office

Office européen des brevets

(19)

(11) **EP 0 504 380 B1**

## (12) EUROPEAN PATENT SPECIFICATION

(45) Date of publication and mention
of the grant of the patent:
**12.11.1997 Bulletin 1997/46**

(21) Application number: **91919319.3**

(22) Date of filing: **17.09.1991**

(51) Int Cl.6: **G01N 33/84**, G01N 27/416

(86) International application number:
**PCT/US91/06719**

(87) International publication number:
**WO 92/06383 (16.04.1992 Gazette 1992/09)**

(54) **METHOD FOR DETERMINATION OF TOTAL CALCIUM EMPLOYING A DILUENT**

VERFAHREN ZUR BESTIMMUNG VON GESAMTEM KALZIUM UNTER VERWENDUNG EINES
VERDÜNNUNGSMITTELS

PROCEDE DE DETERMINATION DE CALCIUM TOTAL EMPLOYANT UN DILUANT

(84) Designated Contracting States:
**AT BE CH DE DK ES FR GB GR IT LI LU NL SE**

(30) Priority: **03.10.1990 US 592768**

(43) Date of publication of application:
**23.09.1992 Bulletin 1992/39**

(73) Proprietor: **BECKMAN INSTRUMENTS, INC.
Fullerton, CA 92634 (US)**

(72) Inventor: **SHU, Frank, R.
La Habra Heights, CA 90631 (US)**

(74) Representative: **Ede, Eric
Fitzpatricks,
4 West Regent Street
Glasgow G2 1RS, Scotland (GB)**

(56) References cited:
**EP-A- 0 166 944        EP-A- 0 203 334
EP-A- 0 304 151        EP-A- 0 319 863
WO-A-89/12827         DE-A- 2 147 066
FR-A- 2 506 296        SU-A- 969 672**

• **Methods of Enzymology, vol.XIII, p.81-90, (1969)**

Remarks:
The file contains technical information submitted
after the application was filed and not included in this
specification

**Description**

## FIELD OF THE INVENTION

The present invention is related to indirect potentiometric methodologies and diluents and particularly to a diluent useful in the indirect potentiometric determination of total calcium in clinical samples of bodily fluids.

## BACKGROUND OF THE INVENTION

Calcium, the fifth most abundant element in the human body, plays an important physiological role and is essential for both the functional integrity of the nervous and muscular systems as well as normal cardiac function. Calcium is also one of the factors that operates in the mechanisms involved in the coagulation of the blood. More than 90% of the calcium in the human body is in the skeleton as calcium phosphate and calcium carbonate -- the remainder of calcium is present in plasma. Plasma calcium levels vary from between about 9.0 and about 11.0mg/100ml. On average, about 46% is bound to protein; slightly more is free and ionized; and the remainder is freely defusable but complexed and ionized. Total calcium, or "CaT", is the combined value of bound calcium ("CaB") free calcium ("CaF" or "Ca++") and protein complexed calcium ("CaP"), or:

$$CaT = CaB + CaF + CaP$$

CaF is generally accepted as being the physiologically active form of calcium in sera. Potentiometric sensors in the form of ion selective electrodes are useful in the determination of CaF.

Ion selective electrode technology involves the use of a reference electrode and an ion selective electrode ("ISE") separated by a membrane which are simultaneously immersed in a sample of a solution containing the desired ion. This simultaneous immersion leads to a potential across the membrane between the electrodes, which potential is proportional to the presence of the desired ion. Most often the investigator desires to only measure the concentration of one ion out of many different ions in solution. Thus, the composition of the ion selective electrode ISE must be capable of transporting the desired ion across the membrane in preference to all other ions which may be present. Two methodologies are associated with ISE technology: direct, where the sample is analyzed directly; and indirect, where the sample is diluted prior to analysis.

Conventional calcium ISE's only measure the free form of calcium. Accordingly, calcium indirect ISE methodologies employ diluents to displace the protein bound to calcium in an attempt to obtain an accurate reading of free calcium. Such conventional methodologies typically make use of strongly acidic diluents prior to ISE measurement in order to denature proteins bound to the calcium, that is, to displace or, "release", the bound calcium[1]. However, some ions, such as citrate (normally present in human sera at a level of about 0.1 mmol/L but during blood transfusions can be present in levels up to about 5 mmol/L), remain effective in binding calcium even under such acidic conditions[2]. Additionally, certain calcium ionophores will not function properly under such acidic conditions. Other methodologies have been described for releasing bound calcium[3]'[4],[5],[6].

EP-A-0 304 151 describes a method for determining the concentrations of total calcium and at least one monovalent ion, e.g. sodium, in a sample which may contain protein-bound calcium wherein the method includes the steps of mixing the sample with a diluent of pH 6.5 - 7.0 including 2-amino-2-hydroxymethyl-1,3 propanediol phosphate but excluding the monovalent ion, and contacting the diluted sample with ion-selective electrodes respectively specifically responsive to calcium ions and the monovalent ion(s) to determine the concentrations of each ion.

Because of the problems associated with denaturing proteins bound to calcium in clinical samples, a diluent that does not rely upon denaturation and that does not utilize conditions which have an adverse affect upon the calcium ISE, as well as analytical methodologies employing such diluents, is desirable and would be of great value in the determination of total calcium in a clinical sample.

## SUMMARY OF THE INVENTION

The present invention as defined in the claims satisfies these requirements. The invention discloses a method for determining the concentration of total calcium in a clinical sample containing protein-bound calcium, the method including the steps of: (a) admixing the sample with a diluent having effective amounts of a pH buffer and at least two agents capable of complexing free calcium in the sample, where the resultant agent-calcium complex association constants ("log K" in base 10 ) each have a range from about 1.5 to about 7.0; and (b) contacting an aliquot of the diluted sample with a calcium-specific ion selective electrode. The response of the calcium-specific ion selective electrode is an indication of the concentration of total calcium in the sample.

Preferably, only two complexing agents are used; most preferably these are dibasic phosphate ($HPO_4$) and citrate ( $(HO)C_3H_4(CO_2)_3$ ). In a particular embodiment, the pH buffer in the diluent can be a phosphoric acid comprising approximately equal molar amounts of monobasic phosphate ($H_2PO_4$) and dibasic phosphate ($HPO_4$), whereby the pH of the diluent is about 7.0. Most preferably, the pH buffer is the above-described phosphoric acid in that the dibasic phosphate portion thereof also serves as one of the complexing agents. The pH of the diluent is preferably within the range of pH 5.0 to pH 8.5; most preferably, the pH of the diluent is about pH 7.0. The concentration of the pH buffer in the diluent is at least about 0.1 mole per liter and the concentration of the complexing agents in the diluent are both at least about 0.002 moles per liter. The diluent can further comprise other optional ingredients such as preservatives and surfactants. The volume to volume ratio of the clinical sample to the diluent is between 1:10 and 1:40, and most preferably this volume to volume ratio is about 1:20.

Because the diluent does not include harsh compounds to denature proteins bound to the calcium, the diluent can be used in association with conventional and commercially available calcium-specific ion selective electrodes.

## DETAILED DESCRIPTION OF A PREFERRED EMBODIMENT

The diluent for measuring total calcium in a clinical sample includes therein effective amounts of at least two agents capable of complexing free calcium in the clinical sample. As used herein, the term "complexing agent" is synonymous with "metal ion buffer". In order to be effective, the resultant agent-calcium complex association constant ("log K" in base 10) has a range of from 1.5 to 7.0; most preferably, this range is from 2.0 to 4.0.

Association constants can be derived from the stability constant ("K") of the complexing agent; the stability constants for a variety of such agents can be readily obtained from published sources. See, for example, Sillen & Martell, <u>Stability Constants Special Publication No. 17</u> London: The Chemical Society, Burlington House, London, England (1964), and Sillen & Martell, <u>Stability Constants, Supplement No. 1 Special Publication No. 25</u> London: The Chemical Society, Burlington House, London England (1971). For example, a measured association constant for calcium-dibasic phosphate is 2.74; a measured association constant for calcium-citrate is 3.60.

Dilution of a clinical sample, e.g., serum, plasma, whole blood, urine, cerebro spinal fluid, with a diluent including therein at least two agents capable of complexing free calcium, equilibrates the protein and other clinical sample compounds bound to the calcium (collectively "CaP") and the agents bound to the calcium ("CaA" and "CaA'") as follows:

$$Ca^{++} + A \quad CaA$$

$$Ca^{++} + A' \quad CaA'$$

$$Ca^{++} + P \quad CaP$$

For convenience, charge symbols for A, A' and P are not included. Under the above criteria, total calcium concentration of the diluted sample for two complexing agents can be defined as:

$$[Ca^{++}] + [CaA] + [CaA'] + [CaP].$$

Accordingly, at equilibrium, the mole fraction ("R") of the free calcium ion can be expressed as follows:

$$R = \frac{[Ca^{++}]}{[Ca^{++}] + [CaA] + [CaA'] + [CaP]} \tag{1}$$

It is to be understood that additional agents can be utilized such that each additional agent (e.g. CaA") is similarly represented in Equation 1.

Equation (1) reduces to:

$$R = \frac{1}{1 + \dfrac{[CaA]}{[Ca^{++}]} + \dfrac{[CaA']}{[Ca^{++}]} + \dfrac{[CaP]}{[Ca^{++}]}} \tag{2}$$

By choosing agents that satisfy the relationship:

$$\frac{[CaA]}{[Ca^{++}]} + \frac{[CaA']}{[Ca^{++}]} + ... >> \frac{[CaP]}{[Ca^{++}]} \tag{3}$$

Equation 2 reduces to

$$R = \frac{1}{1 + \dfrac{[CaA]}{[Ca^{++}]} + \dfrac{[CaA']}{[Ca^{++}]} + ...} \tag{3a}$$

In effect, the agents capable of complexing with free calcium are used to mediate or "control" the mole fraction of free calcium such that the amount of protein-bound calcium becomes inconsequential, as indicated in Equations 3 and 3a.

R can be expressed as a percentage of free calcium, i.e., R x 100%, and [100% - (R x 100%)] is defined as the value of the total mole percentages of the agent-calcium complexes, assuming that Equation 3 is satisfied. It is useful to first determine a value for one of the agent-calcium complexes such that the other agent-calcium complex(es has that same value, such that $\frac{[CaA]}{[Ca^{++}]} = \frac{[CaA']}{[Ca^{++}]}$ However, these values need not be equal; the critical requirement is that Equation 3 is satisfied when the values for the agent-calcium complexes are inserted into Equation 3. As such, less complexing agent can be added to the diluent than the amount actually calculated as long as the lesser amount would continue to satisfy Equation 3.

Additionally, it is useful to obtain values for $\frac{[CaA]}{[Ca^{++}]}$ and $\frac{[CaA']}{[Ca^{++}]}$ such that when incorporated into Equation 3a, R is a relatively small value, the proviso being that if R has too small a value, the calcium concentration of the sample can be difficult to measure with a calcium specific ion selective electrode and if R has too great a value, the agents become ineffective in maintaining a constant mole fraction of free calcium.

The selection of the range of R values is directly related to the volume to volume ratio of the sample to the diluent. For a 1:20 dilution ratio, R is preferably within the range of from 0.009 to 0.03, more preferably within the range of from .01 to .02, and most preferably about .012. As the dilution ratio decreases, the value range for R will decrease, and as the dilution ratio increases, the value range for R will increase. Thus, for a dilution ratio of 1:10, R is preferably within the range of from .0025 to .025; for a dilution ratio of 1:40, R is preferably within the range of from .01 to 0.10.

For a 1:20 dilution ratio, and utilizing a most preferred value for R of .012, Equation 2 can be expressed as follows:

$$.012 = \frac{1}{1 + \dfrac{[CaA]}{[Ca^{++}]} + \dfrac{[CaA']}{[Ca^{++}]} + \dfrac{[CaP]}{[Ca^{++}]}}$$

As previously noted, Equation 2 can be rewritten as Equation 3a when Equation 3 is satisfied. For most clinical samples, it is laborious to determine a value for $\frac{[CaP]}{[Ca^{++}]}$ because all of the stability constants for all proteins and other compounds that bind to calcium in the sample are tedious and/or difficult to determine. However, because the complexing agents are used to control the mole fraction of free calcium, a value for $\frac{[CaP]}{[Ca^{++}]}$ can, for practical purposes, have an assigned approximation value of less than or equal to 1.0. With respect to serum, this value can be empirically estimated using Human Serum Albumin, which is the dominant protein species in serum.

Because Human Serum Albumin ("HSA") is the dominant protein species in serum, a value for $\frac{[CaP]}{[Ca^{++}]}$ can be equated with a value for $\frac{[CaHSA]}{[Ca^{++}]}$. At normal serum levels, HSA has a concentration of approximately 0.6 mmole/L. Assuming a 1:20 dilution ratio, and using the association constant model for the calcium - complex in human serum set forth in Fogh-Anderson, N. "Albumin/calcium association at different pH, as determined by potentiometry" <u>Clin. Chem. 23</u>:2122-2126 (1977),

$$\frac{[CaHSA]}{[Ca^{++}]} = 0.3 \; .$$

Accordingly, when

$$\frac{[CaA]}{[Ca^{++}]} + \frac{[CaA']}{[Ca^{++}]} >> 0.3,$$

Equation 3a is utilized to determine the concentration of the agents to be added to the diluent. Assuming R to be equal to .012, and further assuming that $\frac{[CaA]}{[Ca^{++}]}$ and $\frac{[CaA']}{[Ca^{++}]}$ then

$$0.012 = \frac{1}{1 + (2x)}$$

$$0.012 + .012(2x) = 1$$

$$012(2x) = .988$$

$$x = 41$$

Accordingly,

$$\frac{[CaA]}{[Ca^{++}]} = \frac{[CaA']}{[Ca^{++}]} = 41$$

These values satisfy Equation 3 (82 >> 0.3). Because the value "82" is much greater than 0.3, it is possible to use less of each agent or less of one agent whereby Equation 3 is still satisfied. From a commercial standpoint, this is of beneficial value in that for a particularly strong calcium complexing agent, that is, one having a stability constant indicative of a strong calcium complexing agent, less of the agent can be used without compromising the importance thereof in the diluent.

To determine a useful concentration of the agents ([A] and [A']), the stability constants (K) are utilized as follows:

$$K = \frac{[CaA]}{[Ca^{++}] \times [A]}$$

$$K \times [A] = \frac{[CaA]}{[Ca^{++}]}$$

$$[A] = \frac{\dfrac{[CaA]}{[Ca^{++}]}}{K}$$

As set forth above, for $\frac{[CaA]}{[Ca^{++}]} = 41$, then:

$$[A] = \frac{41}{K}$$

The association constant of the agent-calcium complex is preferably within the range of about 1.5 to about 7.0, with a most preferred range of from about 2.0 to about 4.0. Calcium-dibasic phosphate, at pH 7.0, has a measured association constant of 2.74; calcium-citrate, at pH 7.0, has a measured association constant of 3.60. Accordingly

$$[\text{Dibasic phosphate}] = \frac{41}{10^{2.74}} = 0.075M$$

$$[\text{Citrate}] = \frac{41}{10^{3.60}} = .010M$$

The molar value for dibasic phosphate represents only one-half of the necessary amount of phosphoric acid that must be added to the diluent in that at pH 7.0, the other form of phosphoric acid is monobasic phosphate. Therefore, at pH 7.0, 0.15M phosphoric acid is utilized because of this factor. Additionally, and for the reasons noted above, less citrate can be used under these conditions, for example, .005M (5 mmol).

Examples of suitable agents that satisfy the agent-calcium complex association constant requirements include: dicarboxylic acids; tricarboxylic acids; iminodiacetic acids; sulfonic acids; and organophosphoric acids. Specific agents include: aspartic acid; gluconic acid; succinic acid; oxaloacetic acid; propane-1,2,3-tricarboxylic acid; citric acid; iminodiacetic acid; N-methyliminodiacetic acid; 4,5-dihydroxy-1,3-benzenedisulfonic acid; glycerol-2-phosphate, disodium salt; N-(2-hydroxycyclohexyl) iminodiacetic acid; nitrilotriacetic acid; N'-(2-hydroxyethyl) ethylenediamine-N,N, N'-triacetic acid; ethylenediamine-N,N-diacetic acid; 8-hydroxyquinoline-5-sulfonic acid; and phosphoric acid.

The pH buffer is chosen such that the pH of the diluent is within the range of pH 5.0 to pH 8.5. Preferably, the pH of the diluent is about 7.0. Combinations of pH buffers can be utilized to satisfy these requirements. The pH buffer can comprise at least one of the following: acetic acid; 2-(N-morpholino)ethanesulfonic acid; 3-(N-morpholino) propanesulfonic acid; N-2-hydroxyethyl piperazine-N'-2-ethanesulfonic acid; phosphoric acid; N-[tris-(hydroxymethyl)methyl]glycine; diethynol amine; dimethylamine; phosphoric acid; and the alkyl amines. Most preferably the pH buffer is the aforementioned phosphoric acid. The pH buffer has a concentration in the diluent of at least about 0.1 mole per liter, more preferably a concentration in the diluent of between about 0.1 mole per liter and about 0.4 mole per liter, and most preferably a concentration in the diluent of about 0.15 mole per liter. Other ingredients can be added to the diluent that do not affect the diluent and which are used in association with the pH buffer to balance the charge thereof. For example, when phosphoric acid is used as a pH buffer, it is useful to include in the diluent a "counter cation", i.e. an ingredient to balance the anionic charge of the phosphoric acid. For phosphoric acid, a suitable counter cation is tris-(hydroxymethyl)aminomethane (hereinafter "TRIS").

The diluent can also include therein a surfactant, the preferred surfactant being non-cationic, and more preferably 2,4,7,9-tetramethyl-5-decyn-4,7-diol. The foregoing surfactant is commercially available under the brand-name Surfynol 104™ (Air Products, Allentown, PA). A preservative can also be added to the diluent, such as, for example, phenoxyethanol.

An embodiment of the presently claimed diluent would include the following:

| Total Calcium Diluent | |
| --- | --- |
| Ingredient | Concentration |
| TRIS | 0.30 mole per liter |
| phosphoric acid | 0.15 mole per liter |
| citrate (citric acid) | 5 milimole per liter |
| 2,4,7,9-tetramethyl-5-decyn-4,7, diol | 0.02 per cent by weight |
| phenoxyethanol | 0.02 per cent by weight |
| pH of diluent | 7.0 |

It is to be understood that the foregoing description or an embodiment of the diluent is not to be construed as an indication that the complexing agents are limited to dibasic phosphate and citrate, or that the pH buffer and at least one of the complexing agents are the same. The preceding methodology can be advantageously utilized to determine other agents capable of complexing free calcium which can be added to the diluent for the analysis of clinical samples.

## EXAMPLES

The following examples are presented for illustration purposes only and are not intended to limit the scope of the invention.

Analysis of various diluents for the determination of total calcium was performed on a modified SYNCHRON® ELISE™ analyzer (Beckman Instruments, Inc., Brea, CA.), utilizing a calcium-specific ion selective electrode similar to that described in Reference 1, infra. Serum samples from healthy individuals were diluted one part sample volume to twenty parts diluent; dilution was automatically accomplished by the aforementioned analyzer prior to analysis. All chemicals used for testing were ACS grade, and were freshly prepared prior to analysis. Calcium standards were prepared from a stock solution including therein SRM 915™ calcium carbonate.

Example I

Protein Effect on Calcium Recovery

The aforementioned serum pools were spiked with 5.0 g/dl Human Serum Albumin (Cohn Fraction V, Sigma Chemical Co., St. Louis, MO.) for analysis of protein effect on calcium recovery. Differing amounts of the aforementioned calcium carbonate were added to the protein spiked serum samples. Comparisons were made between two diluents similar to that set forth as an embodiment of the diluent: the first included as a complexing agent only 0.15M phosphoric acid ("PA") and the second including both 0.15M phosphoric acid and 5 mmol citric acid ("PA/CA"). Normalized calcium recovery results (normalized against the calcium recovery values in the absence of HSA) derived from the indirect potentiometric analysis of the samples are presented in Table I:

TABLE I

| Normalized Calcium Recovery | | |
|---|---|---|
| | Diluent Agent | |
| $Ca^{++}$ (mg/dl) | PA | PA/CA |
| 5 | .8667 | 1.0185 |
| 10 | .8713 | 0.9907 |
| 15 | .8782 | 0.9811 |

The results indicate that calcium recovery is significantly improved with the addition of citrate in the diluent.

EXAMPLE II

Citrate Effect on Calcium Recovery

Reference 1, infra, describes a 30% reduction in the recovery of total calcium with the presence of 1 mmol of citrate in serum samples. Increasing amounts of citrate were added to serum samples and the aforementioned diluents of Example I were tested. Normalized calcium recovery values are set forth in Table II:

TABLE II

| Normalized Calcium Recovery | | |
|---|---|---|
| | Diluent Agent | |
| Citrate (mmol) | PA | PA/CA |
| 0 | 1.00 | 1.00 |
| 1.0 | 1.00 | 1.00 |
| 5.0 | .97 | .99 |
| 10.0 | .93 | .97 |

The results set forth in Table II indicate that at the 1.0 mmol citrate level, complete calcium recovery was obtained when either of the tested diluents were utilized. However, at the 5.0 mmol citrate level, the citrate effect on calcium recovery was approximately 3% when only phosphoric acid was used as the complexing agent, while the citrate effect was significantly decreased when both phosphoric acid and citric acid were used as complexing agents. At the 10.0 mmol level, the citrate effect on calcium recovery was reduced by over 50% when both complexing agents were added to the diluent, compared to when only the phosphoric acid was added to the diluent.

EXAMPLE III

Correlation Study

Serum correlation studies were conducted between the indirect potentiometric analysis for total calcium recovery and the atomic absorption method for calcium recovery, using a Jerrel Ash Model 12E™ AA Spectrophotometer. For the indirect potentiometric analysis, the diluent included both of the aforementioned agents, phosphoric acid and citric acid. Correlation results summarized as follows:

$$Y_{Indirect} = 1.0359 * X_{AA} - 0.3302$$

$$N = 97$$

$$R^2 = .9844$$

EXAMPLE IV

Determination of Calcium Concentration

Dilution of a clinical sample can effectively place the diluted sample-calcium concentration into the non-linear range of the calcium-specific ion selective electrode. As such, the diluent preferably includes therein calcium or a salt thereof in an amount effective to correct for a decrease in sensitivity of a calcium-specific ion selective electrode. This additional calcium or calcium salt is referred to as a "spiking factor" or "SF". Generally, and because of the objective thereof, the concentration of the SF added to the sample is relatively small, usually on the order of from 0.015 mmol/L of diluent to 0.035mml/L of diluent, and most preferably, about 0.02 mmol of calcium carbonate is added per liter of diluent.

When the diluent incorporates the SF therein, determination of calcium concentration in the clinical sample can be determined using the following modified Nicolskii-Eisenman equation:

$$C_{Ca} = antilog\ [(E_{Ca} - E^{\circ})/S] - SF \qquad (5)$$

where: $E_{Ca}$ is the value (in mV) of the calcium-specific ion selective electrode towards the calcium in the diluted sample; $E^{\circ}$ is a constant EMF difference (temperature dependent); S is the slope and the electrical response function; SF is the value of the amount of the spiking factor added to the diluent as well as a system "carry-over" value attributed to residual calcium which may remain on the calcium specific ion selective electrode or flow cell; and $C_{Ca}$ is the determined concentration of calcium in the clinical sample. If a SF is not utilized in the diluent, then the concentration of calcium in the clinical sample can be determined as follows:

$$C_{Ca} = antilog\ [(E_{Ca} - E^{\circ})/S)] \qquad (6)$$

where the foregoing definitions apply.

For a preferred analysis of clinical samples for the determination of calcium therein, an automatic electrolyte system utilizing an indirect potentiometric methodology can be utilized, such as, for example, a SYNCHRON® EL-ISE™ analyzer (Beckman Instruments, Inc., Brea, CA.) although the invention is not to be limited in its applicability to this preferred analyzer.

Most preferably, a 50µl clinical sample (e.g. serum) is diluted 20 fold with the most preferred diluent. For the analysis of the diluted sample, the aforementioned analyzer measures the calcium-specific ion selective electrode response this value is represented as $E_{Ca}$. Equations 5 or 6 can then be utilized to determine the value for $C_{Ca}$.

The results from Examples I to IV establish the advantages derived from utilization of at least two calcium complexing agents in a diluent for use in the indirect potentiometric analysis of clinical samples for the determination of total calcium.

The above examples are of preferred embodiments of the disclosed invention.

**BIBLIOGRAPHY**

1. Anker, P. et al. "Neutral Carrier Based Ion-Selective Electrode for the Determination of Total Calcium in Blood Serum." Anal. Chem. 53:1970-1974 (1981).

2. Gawoski, J.M. and Walsh, D. "Citrate Interference in Assays of Total Calcium in Serum." Clin. Chem. 35: 2140-2141 (1989)

3. U.S. Patent No. 4,724,216

4. U.S. Patent No. 3,934,977

5. U.S. Patent No. 4,363,633

6. U.S. Patent No. 4,870,024

## Claims

1. A method for determining the concentration of total calcium in a clinical sample comprising the steps of:

   a) admixing said sample with a diluent to form a diluted sample, said diluent comprising:

      i) a pH buffer for maintaining the pH of the diluent within the range of from 5.0 to 8.5; and
      ii) at least two different complexing agents capable of forming an agent-calcium complex, said agent-calcium complex association constants having a range of from 1.5 to 7.0 wherein said complexing agents are selected from dicarboxylic acids; tricarboxylic acids; iminodiacetic acids; sulfonic acids; organo-phosphoric and phosphoric acids and the total amount of said complexing agents is sufficient to provide said diluted sample with a constant free calcium mole fraction of from 0.0025 to 0.1;

   b) contacting an aliquot of said diluted sample with a calcium specific ion selective electrode; and
   c) measuring the response of said calcium specific ion selective electrode as an indication of the concentration of total calcium in said sample.

2. A method according to claim 1 wherein the volume to volume ratio of said sample to said diluent is from 1:10 to 1:40.

3. A method according to claim 1 wherein the volume to volume ratio of said sample to said diluent is about 1:20.

4. A method according to claim 1 wherein said diluent further includes additives selected from preservatives and surfactants.

5. A method according to claim 1 wherein the pH buffer and one of the complexing agents are the same ingredient.

6. A method according to claim 5 wherein said ingredient is phosphoric acid having molar concentrations of phosphate [monobasic] and phosphate[dibasic] that are substantially the same.

7. A method according to claim 6 further including therein an effective amount of a counter cation.

8. A method according to claim 7 wherein said counter cation is tris(hydroxymethyl)aminomethane.

9. A method according to claim 8 wherein the concentration of said phosphoric acid in said diluent is about 0.15 mole per liter and the concentration of said tris(hydroxymethyl)-aminomethane in said diluent is about 0.30 mole per liter.

10. A method according to claim 1 wherein the pH range of said diluent is about 7.

11. A method according to claim 1 wherein the pH buffer is selected from acetic acid; 2-(N-morpholino)-ethane-sulfonic acid; 3-(N-morpholino) propanesulfonic acid; N-2-hydroxyethyl piperazine-N'-2-ethanesulfonic acid; phosphoric acid; N-[tris-(hydroxymethyl)methyl]-glycine; diethanolamine; dimethylamine; and the alkyl amines.

12. A method according to claim 1 wherein the association constant values of said agent-calcium complexes are each from 2.0 to 4.0.

13. A method according to claim 1 wherein said complexing agents are selected from: aspartic acid; gluconic acid; succinic acid; oxaloacetic acid; propane-1,2,3-tricarboxylic acid; citric acid; iminodiacetic acid; N-methyliminodiacetic acid; 4,5-dihydroxy-1,3-benzene-disulfonic acid; glycerol-2-phosphate, disodium salt; N-(2-hydroxycyclohexyl)iminodiacetic acid; nitrilotriacetic acid; N'-(2-hydroxyethyl)ethylenediamine-N,N,N'-triacetic acid; ethylenediamine-N, N-diacetic acid; 8-hydroxy-quinoline-5-sulfonic acid; and phosphoric acid (dibasic).

**14.** A method according to claim 1 wherein the concentration of said pH buffer in said diluent is at least about 0.1 mole per liter.

**15.** A method according to claim 1 wherein the concentration of said pH buffer in said diluent is from 0.1 mole per liter to 0.4 mole per liter.

**16.** A method according to claim 1 wherein said diluent comprises in effective amounts;

  (a) phosphoric acid; and
  (b) citric acid

wherein the pH of said diluent is from 5.0 to 8.5.

**17.** A method according to claim 16 wherein the concentration of phosphoric acid in said diluent is about 0.15 mole per liter and the concentration of citric acid in said diluent is about 0.005 mole per liter.

**18.** A method according to claim 1 wherein said pH buffer is phosphoric acid, said phosphoric acid comprising molar concentrations of phosphate[monobasic] and phosphate[dibasic] that are substantially the same.

**Patentansprüche**

**1.** Verfahren zur Bestimmung der Konzentration von Gesamt-Kalzium in einer klinischen Probe, mit den folgenden Schritten:

  a) Mischen der Probe mit einem Verdünnungsmittel zur Bildung einer verdünnten Probe, wobei das Verdünnungsmittel:

  i) einen pH-Puffer zum Halten des pH-Wertes des Verdünnungsmittels in dem Bereich von 5,0 bis 8,5; und
  ii) zumindest zwei unterschiedliche Komplexbildner umfaßt, die einen Komplexbildner-Kalzium-Komplex bilden können, wobei die Komplexbildner-Kalzium-Komplex-Assoziationskonstanten einen Bereich von 1,5 bis 7,0 aufweisen, worin die Komplexbildner aus Dicarbonsäuren; Tricarbonsäuren; Imino-Diessigsäuren; Sulfonsäuren; Organo-Phosphor- und Phosphor-Säuren ausgewählt sind und die Gesamtmenge der Komplexbildner ausreicht, um der verdünnten Probe eine konstante freie Kalzium-Mol-Fraktion von 0,0025 bis 0,1 zu erteilen,

  b) In-Berührung-bringen einer Teilmenge der verdünnten Probe mit einer kalzium-spezifischen, ionenselektiven Elektrode und
  c) Messung des Ansprechverhaltens der kalzium-spezifischen, ionenselektiven Elektrode als Anzeige der Konzentration des Gesamtkalziums in der Probe.

**2.** Verfahren nach Anspruch 1,
bei dem das Volumen-zu-Volumen-Verhältnis der Probe zu dem Verdünnungsmittel von 1 : 10 bis 1 : 40 reicht.

**3.** Verfahren nach Anspruch 1,
bei dem das Volumen-zu-Volumen-Verhältnis der Probe zu dem Verdünnungsmittel ungefähr 1 : 20 ist.

**4.** Verfahren nach Anspruch 1,
bei dem das Verdünnungsmittel weiterhin Additive einschließt, die aus Konservierungsmitteln und oberflächenaktiven Mitteln bestehen.

**5.** Verfahren nach Anspruch 1,
bei dem der pH-Puffer und einer der Komplexbildner den gleichen Bestandteil bilden.

**6.** Verfahren nach Anspruch 5,
bei dem der Bestandteil Phosphorsäure mit Molkonzentrationen von Phosphat (einbasisch) und Phosphat (zweibasisch) ist, die im wesentlichen gleich sind.

**7.** Verfahren nach Anspruch 6,
das weiterhin eine wirksame Menge eines Gegenkations einschließt.

**8.** Verfahren nach Anspruch 7,
bei dem das Gegen-Kation Tris(hydroxymethyl)-Aminomethan ist.

**9.** Verfahren nach Anspruch 8,
bei dem die Konzentration der Phosphorsäure in dem Verdünnungsmittel ungefähr 0,15 Mol pro Liter und die Konzentration des Tris(hydroxymethyl)-Aminomethan in dem Verdünnungsmittel ungefähr 0,30 Mol pro Liter beträgt.

**10.** Verfahren nach Anspruch 1,
bei dem der pH-Bereich des Verdünnungsmittels ungefähr 7 ist.

**11.** Verfahren nach Anspruch 1,
bei dem der pH-Puffer aus Essigsäure, 2-(N-Morpholino)-Äthan-Sulfonsäure; 3-(N-Morpholino)-Propansulfonsäure; N-2-Hydroxyäthyl-Piperazin-N'-2-Äthansulfonsäure; Phosphorsäure; N-[Tris-(Hydroxymethyl)methyl]-Glyzin; Diäthanolamin; Dimethylamin; und den Alkylaminen ausgewählt ist.

**12.** Verfahren nach Anspruch 1,
bei dem die Assozierungs-Konstantenwerte der Komplexbildner-Kalzium-Komplexe jeweils von 2,0 bis 4,0 betragen.

**13.** Verfahren nach Anspruch 1,
bei dem die Komplexbildner ausgewählt sind aus: Asparaginsäure; Glukonsäure; Bernsteinsäure; Oxal-Essigsäure; Propan-1,2,3-Tri-Carbonsäure; Zitronensäure; Imino-Diessigsäure; N-Methylimino-Diessigsäure; 4,5-Dihydroxy-1,3-Benzol-Disulfonsäure; Gylzerin-2-Phosphat; Dinatriumsalz; N-(2-Hydroxycyclohexyl)Imino-Diessigsäure; Nitrilo-Triessigsäure; N'-(2-Hydroxyäthyl)Äthylendiamin-N,N,N'-Triacetatsäure; Äthylendiamin-N, N-Diacetatsäure; 8-Hydroxy-Quinolin-5-Sulfonsäure; und Phosphorsäure (zweibasisch).

**14.** Verfahren nach Anspruch 1,
bei dem die Konzentration des pH-Puffers in dem Verdünnungsmittel zumindestens 0,1 Mol pro Liter beträgt.

**15.** Verfahren nach Anspruch 1,
bei dem die Konzentration des pH-Puffers in dem Verdünnungsmittel von 0,1 Mol pro Liter bis 0,4 Mol pro Liter beträgt.

**16.** Verfahren nach Anspruch 1,
bei dem das Verdünnungsmittel in wirksamen Mengen:

(a) Phosphorsäure und
(b) Zitronensäure umfaßt,

worin der pH-Wert des Verdünnungsmittels von 5,0 bis 8.5 reicht.

**17.** Verfahren nach Anspruch 16,
bei dem die Konzentration der Phosphorsäure in dem Verdünnungsmittel ungefähr 0,15 Mol pro Liter ist und die Konzentration der Zitronensäure in dem Verdünnungsmittel ungefähr 0,005 Mol pro Liter ist.

**18.** Verfahren nach Anspruch 1,
bei dem der pH-Puffer Phosphorsäure ist, wobei die Phosphorsäure Molkonzentrationen von Phosphat [einbasisch] und Phosphat [zweibasisch] aufweist, die im wesentlichen gleich sind.

**Revendications**

**1.** Méthode pour déterminer la concentration en calcium total dans un échantillon clinique comprenant les étapes de :

a) mélanger ledit échantillon avec un diluant pour former un échantillon dilué, ledit diluant comprenant :

i) un tampon du pH pour maintenir le pH du diluant entre 5,0 et 8,5; et
ii) au moins deux agents complexants différents capables de former un complexe agents-calcium, les constantes d'association dudit complexe agents-calcium ayant une gamme de 1,5 à 7,0 où lesdits agents complexants sont sélectionnés parmi des acides dicarboxyliques; des acides tricarboxyliques; des acides iminodiacétiques; des acides sulfoniques; des acides organophosphoriques et phosphoriques et la quantité totale desdits agents complexants est suffisante pour produire ledit échantillon dilué avec une fraction molaire constante en calcium libre de 0,0025 à 0,1;

b) mettre en contact une aliquote dudit échantillon dilué avec une électrode sélective d'un ion spécifique du calcium; et
c) mesurer la réponse de ladite électrode sélective d'un ion spécifique du calcium en tant qu'indication de la concentration du calcium total dans ledit échantillon.

2. Méthode selon la revendication 1 où le rapport du volume au volume dudit échantillon audit diluant est de 1:10 à 1:40.

3. Méthode selon la revendication 1 où le rapport du volume au volume dudit échantillon audit diluant est d'environ 1:20.

4. Méthode selon la revendication 1 où ledit diluant contient de plus des additifs sélectionnés parmi des conservateurs et des agents tensio-actifs.

5. Méthode selon la revendication 1 où le tampon du pH et un des agents complexants sont le même ingrédient.

6. Méthode selon la revendication 5 où ledit ingrédient est l'acide phosphorique ayant des concentrations molaires de phosphate (monobasique) et phosphate (dibasique) qui sont sensiblement les mêmes.

7. Méthode selon la revendication 6 contenant de plus une quantité efficace d'un contre cation.

8. Méthode selon la revendication 7 où ledit contre cation est le tris(hydroxyméthyl)aminométhane.

9. Méthode selon la revendication 8 où la concentration dudit acide phosphorique dans ledit diluant est d'environ 0,15 mole par litre et la concentration dudit tris(hydroxyméthyl)aminométhane dans ledit diluant est d'environ 0,30 mole par litre.

10. Méthode selon la revendication 1 où le pH dudit diluant est d'environ 7.

11. Méthode selon la revendication 1 où le tampon du pH est sélectionné parmi l'acide acétique; l'acide 2-(N-morpholino)-éthane-sulfonique; l'acide 3-(N-morpholino) propanesulfonique; l'acide N-2-hydroxyéthyl pipérazine-N'-2-éthanesulfonique; l'acide phosphorique; la N-[tris-(hydroxyméthyl)méthyl]-glycine; la diéthanolamine; la diméthylamine; et les alkyl amines.

12. Méthode selon la revendication 1 où les valeurs des constantes d'association dudit complexe agents-calcium sont comprises entre 2,0 et 4,0.

13. Méthode selon la revendication 1 où lesdits agents complexants sont sélectionnés parmi : l'acide aspartique; l'acide gluconique; l'acide succinique; l'acide oxaloacétique; l'acide propane-1,2,3-tricarboxylique; l'acide citrique; l'acide iminodiacétique; l'acide N-méthyliminodiacétique; l'acide 4,5-dihydroxy-1,3-benzène-disulfonique; le glycérol-2-phosphate, sel disodique; l'acide N-(2-hdyroxycyclohexyl)iminodiacétique; l'acide nitrilotriacétique; l'acide N'-(2-hydroxyéthyl)éthylènediamine-N,N,N'-triacétique; l'acide éthylènediamine-N,N-diacétique; l'acide 8-hydroxy-quinoléine-5-sulfonique; et l'acide phosphorique (dibasique).

14. Méthode selon la revendication 1 où la concentration dudit tampon du pH dans ledit diluant est d'au moins environ 0,1 mole par litre.

15. Méthode selon la revendication 1 où la concentration dudit tampon du pH dans ledit diluant est comprise entre 0,1 mole par litre et 0,4 mole par litre.

16. Méthode selon la revendication 1 où ledit diluant comprend en quantités efficaces;

      (a) de l'acide phosphorique; et
      (b) de l'acide citrique

      où le pH dudit diluant est compris entre 5,0 et 8,5.

17. Méthode selon la revendication 16 où la concentration en acide phosphorique dans ledit diluant est d'environ 0,15 mole par litre et la concentration en acide citrique dans ledit diluant est d'environ 0,005 mole par litre.

18. Méthode selon la revendication 1 où ledit tampon du pH est l'acide phosphorique, ledit acide phosphorique comprenant des concentrations molaires de phosphate [monobasique] et phosphate[dibasique] qui sont sensiblement les mêmes.